# EUROPEAN PATENT SPECIFICATION

(11) **EP 3 368 100 B1**
(45) Date of publication and mention of the grant of the patent: **18.03.2020**
(21) Application number: 16815630.5
(22) Date of filing: 27.10.2016
(51) Int. Cl.: A61M 1/36

(54) **APPARATUS FOR THE REGIONAL ANTICOAGULATION OF THE BLOOD**
VORRICHTUNG ZUR REGIONALEN BLUTGERINNUNGSHEMMUNG
APPAREIL D'ANTICOAGULATION RÉGIONALE DU SANG

(30) Priority: 29.10.2015 IT UB20155007
(43) Date of publication of application: 05.09.2018
(73) Proprietor: O2 Group SA, 6900 Lugano (CH)
(72) Inventor: FONTANA, Michele, 84018 Scarfati (SA) (IT)
(74) Representative: Mincone, Antimo
(86) International application number: PCT/IB2016/001542
(87) International publication number: WO 2017/072576

(56) References cited:
- GB-A- 2 086 925
- US-A1- 2007 066 928

## Description

The present invention relates to an apparatus for the treatment of blood and relates, in particular, a device for regional anticoagulation by subtraction of Ca++ ions suitable for treatment with extracorporeal circulation.

Over the last years the prevailing trend in the medical field and, in particular, in treatments involving the extracorporeal circulation, is to limit the use of heparin as an anticoagulant to eliminate potentially harmful side effects. The use of heparin has effects on the entire body and is therefore defined as "systemic anticoagulation."

The use of means that allow to maintain fluid the blood within an extracorporeal circuit but that does not affect the whole patient's organism is defined as "regional anticoagulation." Among these means the "Calcium-Citrate" system is among the most common.

It consists in adding to the blood taken from the patient, and then very closely to the access point (a catheter or an appropriate needle), a solution containing citrate (generally Citrate Trisodium C₆H₅Na₃O₇) which plays an anticoagulant action, thanks to its capacity to "chelate" or block the calcium ions Ca++.

The aforesaid Calcium ions are indispensable to activate the so-called "coagulative cascade", namely the phenomenon that initiates the coagulation of blood; when the concentration of Ca++ ions falls below a certain value (about 0.4 mmol/L) the blood does not coagulate more.

In regional anticoagulation treatments using this technique, at the opposite end of the circuit, i.e. immediately before the blood in extracorporeal circulation is returned to the patient, a suitable solution containing Calcium (Calcium gluconate or Calcium chloride) is added to the blood itself, with the aim to bring the blood to the natural physiological coagulation conditions, compensating the Calcium which could be lost, or be eliminated along with the liquid removed during the extracorporeal treatment.

The addition of the Calcium solution may also be performed to the patient at a different point from the extracorporeal circuit and through independent means.

This system is mainly adopted in hemofiltration machines (dialysis machines for patients in the acute phase) and also in patients undergoing regular dialysis (chronic).

These machines generally employ peristaltic pumps and employ considerable quantities of dialysis and replacement liquid to perform blood purification.

In practice, the blood can be diluted before and / or after the passage through a hemofilter (or artificial kidney) where the dialysis liquid in countercurrent re-establishes the correct ratio of dissolved substances as well as to eliminate the excess water and the waste substances.

Among the procedures that can be performed using the "Citrate-Calcium" system there are, for example, those known by the acronyms CRRT, SCUF, HF, CVVH, CVVHV, CVVHVHF, CVVHD, CVVHFD, IVVH.

In the case of extracorporeal treatments that do not provide for a removal of fluid from the patient (as in the case of CO2 removal treatments) it has so far made use of systemic anticoagulation (heparin) given the difficulty of eliminating from the patient (substantial) amounts of liquid present in existing solutions for the administration of citrate (also 5 liters per bag) and that are infused also quantity / flows greater than 2,000 ml / h.

The patient who has not a renal failure and is subjected to another extracorporeal treatment such as the extracorporeal removal of CO₂, would be forced to eliminate by means of a natural way (urine) the excess liquid received from the diluted citrate bag; this necessity subjects the kidneys to an excessive workload and could lead to failure in the correct composition of electrolytes and other physiologically substances contained in the patient's blood.

It is important to note that the Citrate which binds to Calcium is still released within the blood circuit and enters the patient's circulatory system where the liver, kidneys and muscles ensure metabolize it to bicarbonate. This involves a considerable workload for the liver in addition to the contraindication to enter large doses of bicarbonate in the human body.

US2007/066928 A1 (Lannoy Jean-Michel, 22.3.2007) discloses an apparatus for regional anticoagulation of blood in an extracorporeal blood circuit. The apparatus comprises a pump and source of citrate with which citrate is added to the arterial line and an electrolyte source and pump connected to the venous line. With the apparatus of US2007/066928 A1 it is necessary to add a substance to the blood to achieve anticoagulation.

The main object of the present invention is to eliminate the above mentioned drawbacks. This result has been achieved according to the invention by adopting the idea of providing an apparatus adapted to retain the Ca++ ions from blood with the features described in claim 1. The apparatus of the invention allows to drastically reduce the Ca++ ions from the blood limited to the section affected by the treatment. Furthermore, the apparatus may allow, in addition, the replenishing of the same ions downstream in the blood flow of the circuit, before the reinfusion to the patient. Other characteristics are described in the dependent claims.

The advantages and features of the present invention will be understood by anyone skilled in the art from the following description and with the help of the attached drawings given as a practical exemplification of the invention, but not to be considered in a limitative sense, wherein:
- Fig. 1 illustrates a possible example of embodiment of the invention, represented schematically and not to scale;
- Fig. 2 is a schematic representation of a particular in order to better illustrate the example of Fig.1;
- Fig. 3 illustrates another possible example of embodiment of the invention, represented schematically and not to scale.

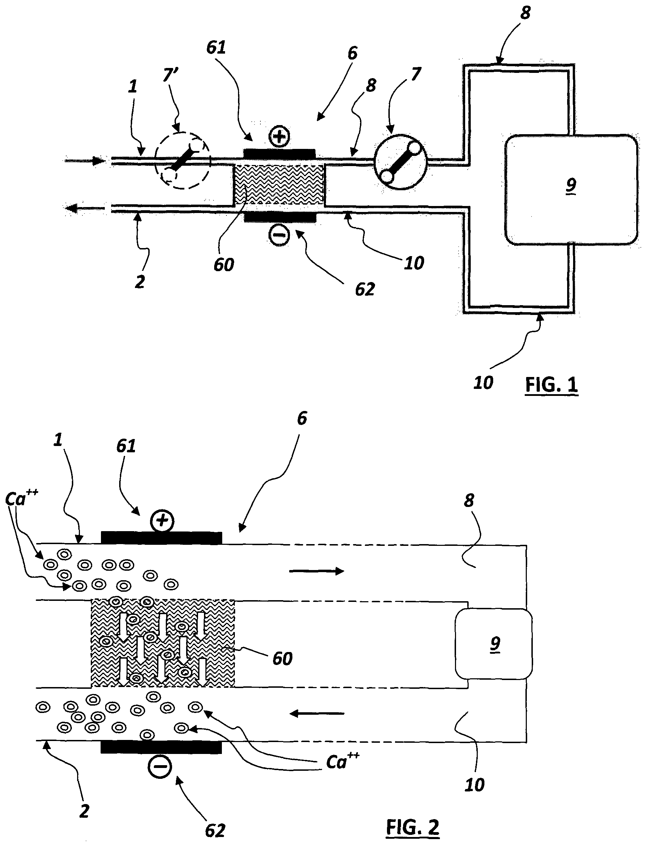

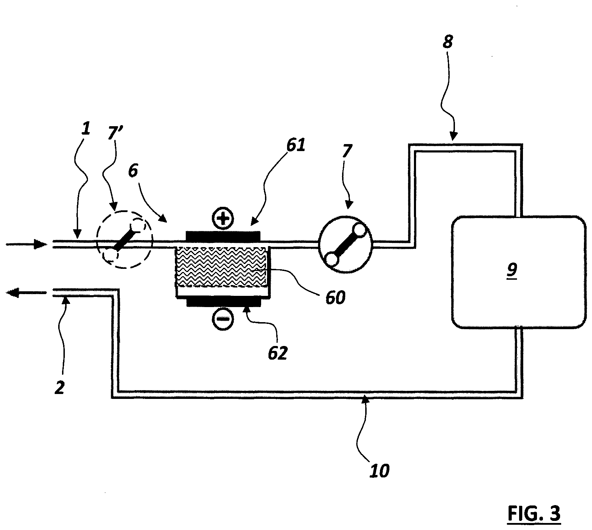

In the present description reference is made to a generic extracorporeal blood treatment with a passage through a hemofilter (marked with the reference 9 in Fig.1 and Fig.3); the hemofilter choice does not exclude that the circuit of the invention, or, more precisely, the extracorporeal circuit to which the present invention can be applied with many advantages, can comprise, for example, in place of or in addition to the hemofilter, an oxygenator for the removal of CO₂, or, in place of the oxygenator or in addition to the same oxygenator, any other component as absorbers of cytokines, filters for cholesterol, cartridges for drug delivery, etc. including a circuit for dialysis or hemofiltration.

With reference to the diagrams of Fig.1 and Fig.3, a circuit made according to the present invention is usable to obtain the regional anticoagulation by retaining or displacing Ca2+ ions, also called Ca++ in the present description.

The invention is advantageously applicable to a blood-circuit which comprises an inlet conduit (1) through which passes the blood coming from the patient; on the conduit (1) it is acting a blood pump (7) adapted to move the blood within the circuit. Downstream of the pump (7), the blood passes through the portion (8) of the blood circuit to be received by the hemofilter (9) where it is submitted to the treatment. Downstream of the hemofilter (9), through the portion (10), the treated blood is returned to the patient through the outlet duct (2).

The circuit (as generally shown in the embodiment of Fig. 3) comprises a device (6) which is substantially composed of a container, preferably made of biocompatible plastic material. Inside the device (6) is disposed a first electrode (61) of a suitable material and a selective membrane (60) permeable to calcium ions Ca++. Such electrode (61), positively charged with a suitable potential difference of the order of a few volts, together with the electrode (62) negatively charged and place beyond the semipermeable membrane (60), convey, by passing them through said membrane semipermeable (60), the ions Ca++, blocking them onto the electrode (62) and then removing them from the blood flow in input and thereby obtaining a regional anticoagulation. (The electrode (62) will be suitably dimensioned according to the blood flow and the expected duration of treatment since it is subject to saturation, once covered by the subtracted ions Ca ++).

A more advanced version of such a device, schematically shown in Fig. 1 and detailed shown in Fig. 2, provides a container through which pass both the blood flows; the flow of the blood flow taken from the patient (which is received by the apparatus 6 through the conduit 1) and which must undergo the extracorporeal treatment (for example, haemofiltration) and the blood return flow to the patient (passing through the duct 2).

In the embodiment of the invention represented in the drawings of Figs. 1 and 2, the housing of the device (6) is divided into two parts in which said two blood flows flow, the same flows being separated by the semipermeable membrane (60) which is selective for Ca++ ions.

A positive electrode (anode) (61) is placed on the wall of the duct (1) opposite the membrane (60), thus acting on the incoming blood, while a corresponding negative electrode (cathode) (62) is symmetrically positioned on the opposite side to the membrane into the compartment (2) of the blood that has undergone extracorporeal treatment and has to be returned to the patient.

Inside this device the blood will flow in contact with the surface of the membrane and calcium ions Ca++ contained in the blood itself, attracted by the cathode (62) will be subtracted from the blood flow in output from the device (6) and, through the selective membrane semipermeable, returned to the blood flow in restitution to the patient, thus restoring the physiological coagulation conditions. This particular arrangement of the electrodes on the wall of the tubes opposite to the semipermeable membrane finds its justification in the fact that, given the relative slowness of the transport speed of the ions through the blood, these ions are carried by the blood stream, after passing through the semipermeable membrane into the duct (2) and then returned to the patient before being captured by the electrode (62).

In other words, the regional anticoagulation regards exclusively the circuit portion comprising the portions (8) and (10), i.e. that part of the circuit in which it is appropriate to have such a blood condition for the treatment performed by the device (9). Downstream of the apparatus (6), in the outlet duct (2) that returns the blood to the patient, the blood has the previous or physiological conditions of Ca++ ions. In the embodiment defined as more evolved, the same apparatus (6) contribute to restore the initial conditions. In the version defined as simplified (and illustrated in Fig.3) the apparatus (6) provides for reducing the amount of Ca++ ions directed to the device (9) for the treatment of blood and the reestablishment of the original or physiological conditions is obtained without the intervention of the apparatus (6).

A suitable software provides to regulate the voltage and the current in two electrodes (61, 62) for adapting it to the blood velocity, providing to interrupt the current in case of arrest blood flow.

The present invention is also applicable in extracorporeal treatments that do not use pumps but which exploit the pressure difference between the artery and vein.

According to the invention, it is obtained the reduction of the concentration of Ca++ ions in the blood in output from the device (6) and the blood is then non-coagulable and therefore suitable to undergo all subsequent extracorporeal treatments without danger of coagulating.

The main advantage of this device over the prior art, is to make the blood not coagulable exclusively within the extracorporeal circuit without use of trisodium citrate which require high dilutions and some metabolism of the compound Calcium-Citrate. In the most advanced version, the ions Ca++ are only temporarily withdrawn from the stream circulating in the extracorporeal circuit, and returned just before the reinfusion into the patient.

Further advantages reside in avoiding the need to infuse liquids in quantity (and consequently to remove them), an operation that has a cost in the diluted Citrate bags and in operator workload. Through an appropriate adjustment of the operating parameters of the electrodes it will be also possible to avoid an excess of removal avoiding the need to compensate for the Calcium ions subtracted (except the fraction of them that remain inevitably linked to the cathode (62)).

The device will be realized in different sizes containing various surfaces and membrane quantities to satisfy the different needs of flow and duration of the various extracorporeal treatments. The membrane (60) can be done in Polyvinyl chloride (PVC) or other material that will be considered suitable. Such a membrane will be subjected to specific chemical treatments which provide a compound referred to as "carrier" which allows the membrane itself, treated with such product to become selectively permeable to Ca++ ions.

Such "carrier" must first be solubilized by a "solvent" to allow the diffusion and the subsequent deposit and bond with the membrane.

Hereinafter will be described some examples of realization of the membrane (60).

The present invention therefore realizes a membrane separation, or a physical process which, thanks to the presence of semi-permeable membranes, allows the crossing in a selective manner on the basis of size and / or charge of the species present in solution.

At the base of the present invention there is also the idea to use a membrane for electrodialysis, i.e. to use an electromotive force to permit the transport of chemical species through the membrane itself. The presence of a potential difference across two metal electrodes determines the migration of cations, positively charged, towards the negative pole, and the migration of the anions, negatively charged, towards the positive pole. Since the membrane (60) is of the selective type, it will allow to transfer only the species in question, i.e. the calcium ion from one side to the other of the same membrane.

The membrane (60) may be constituted by a solid support (cellulose acetate, polyvinyl chloride, cellulose nitrate, etc.), saturated with a viscous hydrophobic solvent that contains an ionophore, i.e. a chemical species "ions carrying", able, however, to selectively bind the Ca2+ ion on the membrane surface and to transport it through it thanks to the mobility provided by hydrophobic solvent in the solid support.

In the embodiment of the membrane, some possible combinations can be formed according to what is listed below.

As the carrier, one of the species that have a marked selectivity in the calcium ion binding, for example:
- (-)-(R,R-N,N'-Bis-[11-(ethoxycarbonyl)undecyl]-N,N',4,5-tetramethyl-3,6-diamide dioxaoctane--, Diethyl N,N'-[(4R,5R)-4,5-dimethyl-1,8-dioxo-3,6-dioxaoctamethylene] bis (12-methylaminododecanoate);
- N,N,N',N'-Tetra[cyclohexyl]diglycolic acid diamide, N,N,N',N'-Tetracyclohexyl-3-oxapentanediamide;
- Didecyl-calcium phosphate;
- Potassium tetrakis (4-chlorophenyl) borate;

As solvent, with the function of allowing the carrier to move within the membrane in the direction imposed by the electric field, for example:
- Bis (1-butylpentyl) decane-1,10-diyl diglutarate;
- 2-Nitrophenyl octyl ether;
- Bis (2-ethylhexyl) phthalate;
- Dioctyl phenyl-phosphonate.

The solid support may be achieved, for example, polyvinyl chloride, cellulose acetate, cellulose nitrate.

The realization of the membrane will be made in appropriate percentages dissolving the PVC, the ionophore and the solvent in tetrahydrofuran, a further solvent that will make the mixture homogeneous and that, as a result of its evaporation, leave a thin layer which will constitute the membrane to be used.

In another embodiment of the invention, illustrated in broken line in Fig.1 and 3, the apparatus (6) is arranged downstream of the pump (7).

In practice, an apparatus (6) according to the present invention it can be used to reduce the amount of Ca++ ions in the blood which has to receive a treatment in a circuit for blood treatment comprising at least one inlet duct (1) for the blood taken from the patient, a device for blood processing (9), an outlet duct for return the treated blood to the patient. The apparatus (6) comprises a membrane (60) made of a material selectively permeable to Ca++ ions.

Furthermore, the apparatus comprises means (61, 62) for determining a difference in electrical potential, positioned and acting in correspondence of the inlet duct (1) of the blood to determine a removal of Ca++ ions from the blood going to the device for the treatment (9). In other words, the blood that reaches the hemofilter (9) (or another device for blood treatment, such as an oxygenator or any other component as absorbers of cytokines, filters for cholesterol, cartridges for drug delivery, etc.. including a circuit for dialysis or hemofiltration) is private drastically of Ca ++ ions thus determining a condition of regional anticoagulation.

Again with reference to the references of the accompanying drawings, the means (6) for determining a difference in electric potential comprise a first electrode or positive electrode (61) arranged in correspondence of said duct (1) in a position facing or opposite to said membrane (60) so as to cross the membrane (60) by the Ca ++ ions (repelled from the electrode (anode) (61) and attracted to the other electrode (cathode) (62). In addition, the membrane (60) is arranged interposed between the inlet duct (1) and the outlet duct (2), said means (6) for determining a difference in electrical potential comprising a second electrode or negative (62) arranged in correspondence of the outlet duct (2) in a position facing or opposite to said membrane (60) so as to attract in said outlet duct (2) the Ca ++ ions that pass through the said membrane (60).

In practice, the membrane (60) is disposed interposed between the inlet duct (1) and said outlet duct (2), determining a communication or by-pass between them to permit the transfer of Ca++ ions from the inlet duct (1) to the outlet duct (2). The part of the device provided with the circuit (9) is therefore by-passed by the Ca++ ions that do not affect the coagulation conditions during the treatment undergone by the blood.

The invention also relates to a circuit for the treatment of blood comprising an apparatus as previously described and claimed below. The circuit object of the invention is therefore characterized in that it comprises a portion in which the blood is deprived of Ca++ ions for the execution of a treatment by means of a corresponding device (9). According to a first version, or simplified version, the apparatus (6) disposed on the circuit can reduce the amount of Ca ++ ions in the blood directed to the treatment; according to a second version, or evolved version, the same apparatus (6) may contribute, at least partially, to the reintegration of Ca++ ions in the blood that is returned to the patient.

From the above description the invention is implemented by a method for the treatment of blood which does not form part of the invention and which provides that the same blood, before being subjected to the processing (hemofiltration and / or oxygenation and / or absorption of cytokines and / or filtering for cholesterol and / or release of drugs and / or dialysis), is deprived of a quantity of Ca++ ions by passage through a semipermeable membrane. These Ca++ ions are replenished when the blood is returned to the patient. The operation of reduction of the amount of Ca++ ions may be accomplished by means adapted to determine a difference in electrical potential. The same means, by acting with opposite polarity, can be used, even partially, for the reintegrating operation.

The description relating to the circuits of the drawings is intended as an example and the advantages of the present invention are applicable to any circuit or apparatus in which the anticoagulation is necessary. The invention is defined by the claims.

## Claims

1. Apparatus for the regional anticoagulation of the blood, usable for reducing the amount of Ca++ ions in the blood which has to receive a treatment in a circuit for the treatment of blood, the apparatus comprising an inlet conduit (1) for the blood collected from the patient, a device for the treatment of the blood (9), an outlet conduit for returning the treated blood to the patient, **characterised in that** the apparatus comprises a membrane (60) made of a material selectively permeable to Ca++ ions.

2. Apparatus according to claim 1, **characterized in that** it comprises means (6) for determining a difference of electric potential, positioned and acting in correspondence of said inlet conduit (1) of the blood to determine a removal of Ca++ ions from the blood directed to said treatment device (9).

3. Apparatus according to claim 2, **characterized in that** said means (6) for determining a difference of electric potential comprise a first electrode or positive electrode (61) arranged in correspondence of said inlet conduit (1) in a position facing or opposite side with respect to said membrane (60) and another negative electrode (62) symmetrical with respect to the membrane (60) and oppositely positioned to said membrane, so as to cross the said membrane (60) by the Ca++ ions.

4. Apparatus according to claim 3, **characterized in that** said membrane (60) is arranged interposed between said inlet conduit (1) and said outlet conduit (2), said means (6) for determining a difference of electric potential provide that the second negative electrode (62) is arranged in correspondence of the outlet conduit (2) in a position facing or opposite to said membrane (60) so as to attract in said outlet conduit (2) the Ca ++ ions that pass through the said membrane (60).

5. Apparatus according to claim 1, **characterized in that** said membrane (60) is arranged interposed between said inlet conduit (1) and said outlet conduit (2), determining a communication between them to permit the transfer of ions Ca ++ from the inlet conduit (1) to the outlet conduit (2).

6. Apparatus according to one of the preceding claims, **characterized in that** the device for blood treatment (9) comprises at least one component of the whole consisting of: hemofilter, oxygenator, cytokines absorbers, filters for cholesterol, cartridges for the release of drugs, circuit for dialysis or hemofiltration.

7. Apparatus according to one of the preceding claims, where the circuit for the treatment of the blood comprises a pump (7), **characterized in that** the apparatus is arranged upstream of the pump (7).

8. Apparatus according to one of claims 1 to 7, where the circuit for the treatment of the blood comprises a pump (7), **characterized in that** the apparatus is arranged downstream of the pump (7).

9. Apparatus according to one of the preceding claims, **characterized in that** the membrane (60) is made from a solid support saturated with a viscous hydrophobic solvent which contains an ionophore or transporter or carrier, in which:
- the ionophore or transporter or carrier is selected from the group comprising:
a. (-)-(R,R)-N,N'-Bis-[11-(ethoxycarbonyl)undecyl]-N,N',4,5-tetramethyl-3,6-diamide dioxaoctane--, Diethyl N,N'-[(4R, 5R)-4,5-dimethyl-1,8-dioxo-3,6-dioxaoctamethylene] bis (12-methylaminododecanoate);
b. N,N,N',N'-Tetra [Cyclohexyl] diglycolic acid diamide, N,N,N',N'-Tetracyclohexyl-3-oxapentanediamide;
c. didecyl-calcium phosphate;
d. Potassium tetrakis (4-chlorophenyl) borate;
- the solvent is selected from the group comprising:
a. Bis (1-butylpentyl) decane-1,10-diyl diglutarate;
b. 2-Nitrophenyl octyl ether;
c. Bis (2-ethylhexyl) phthalate;
d. dioctyl phenyl-phosphonate; and
- the support is selected from the group comprising: polyvinyl chloride, cellulose acetate, cellulose nitrate.

10. Circuit for the treatment of the blood **characterized in that** it comprises an apparatus realized according to one of the preceding claims.

## Patentansprüche

1. Gerät für die lokale Antikoagulation des Bluts, das zum Reduzieren der Menge an Ca++-lonen in dem Blut verwendbar ist, das eine Behandlung in einem Kreislauf für die Behandlung von Blut erhalten soll, wobei das Gerät eine Einlassleitung (1) für das Blut umfasst, das aus dem Patienten gesammelt wird, eine Vorrichtung für die Behandlung des Bluts (9), eine Auhyslassleitung für das Zurückführen des behandelten Bluts zu dem Patienten, **dadurch gekennzeichnet, dass** das Gerät eine Membran (60) umfasst, die aus einem Material besteht, das selektiv für Ca++-lonen durchlässig ist.

2. Gerät nach Anspruch 1, **dadurch gekennzeichnet, dass** es Mittel (6) für das Bestimmen eines Unterschieds des elektrischen Potenzials umfasst, die in Entsprechung mit der Einlassleitung (1) des Bluts positioniert sind und wirken, um ein Entfernen von Ca++-lonen aus dem Blut, das zu der Behandlungsvorrichtung (9) gelenkt wird, zu bestimmen.

3. Gerät nach Anspruch 2, **dadurch gekennzeichnet, dass** die Mittel (6) zum Bestimmen eines Unterschieds des elektrischen Potenzials eine erste Elektrode oder positive Elektrode (61) umfassen, die in Entsprechung mit der Einlassleitung (1) in einer Position eingerichtet ist, die in Bezug auf die Membran (60) zugewandt ist oder auf der entgegengesetzten Seite, und eine andere, negative Elektrode (62), die in Bezug auf die Membran (60) symmetrisch und der Membran entgegengesetzt derart positioniert ist, dass die Membran (60) von den Ca++-lonen durchquert wird.

4. Gerät nach Anspruch 3, **dadurch gekennzeichnet, dass** die Membran (60) zwischen der Einlassleitung (1) und der Auslassleitung (2) eingerichtet eingefügt ist, wobei die Mittel (6) zum Bestimmen eines Unterschieds des elektrischen Potenzials vorsehen, dass die zweite negative Elektrode (62) in Übereinstimmung mit der Auslassleitung (2) in einer Position der Membran (60) zugewandt oder ihr entgegengesetzt eingerichtet ist, um in die Auslassleitung (2) die Ca++-lonen anzuziehen, die durch die Membran (60) durchgehen.

5. Gerät nach Anspruch 1, **dadurch gekennzeichnet, dass** die Membran (60) zwischen der Einlassleitung (1) und der Auslassleitung (2) eingefügt eingerichtet ist, wobei zwischen ihnen eine Verbindung bestimmt wird, um den Transfer von Ca++-lonen von der Einlassleitung (1) zu der Auslassleitung (2) zu erlauben.

6. Gerät nach einem der vorstehenden Ansprüche, **dadurch gekennzeichnet, dass** die Vorrichtung für Blutbehandlung (9) mindestens ein Bauteil des Ganzen umfasst, das aus Folgendem besteht: Hämofilter, Oxygenator, Zytokinenabsorber, Cholesterinfilter, Patronen für die Freisetzung von Arzneimitteln, Kreislauf für Dialyse oder Hämofiltration.

7. Gerät nach einem der vorstehenden Ansprüche, wobei der Kreislauf für die Behandlung des Bluts eine Pumpe (7) umfasst, **dadurch gekennzeichnet, dass** das Gerät stromaufwärts der Pumpe (7) eingerichtet ist.

8. Gerät nach einem der Ansprüche 1 bis 7, wobei der Kreislauf für die Behandlung des Bluts eine Pumpe (7) umfasst, **dadurch gekennzeichnet, dass** das Gerät stromabwärts der Pumpe (7) eingerichtet ist.

9. Gerät nach einem der vorstehenden Ansprüche, **dadurch gekennzeichnet, dass** die Membran (60) aus einem festen Träger hergestellt ist, der mit einem viskosen wasserabstoßenden Lösemittel gesättigt ist, das ein lonophor oder einen Transporter oder einen Träger enthält, wobei:
- der lonophor oder Transporter oder Träger aus der Gruppe ausgewählt ist, die Folgende umfasst:
a. (-)-(R, R)-N,N'-Bis-[11-(ethoxycarbonyl)-undecyl]-N,N',4,5-tetramethyl-3,6-diamiddioxaoctan--, Diethyl N,N'-[(4R, 5R)-4,5-dimethyl-1,8-dioxo-3,6-dioxaoctamethylen] bis (12-methylaminododecanoat);
b. N,N,N',N'-Tetra-[Cyclohexyl]-Diglycolsäurediamid, N,N,N', N'-Tetracyclohexyl-3-oxapentandiamid;
c. Didecyl-Calciumsphosphat;
d. Kalium-Tetrakis-(4-chlorphenyl)-Borat;
- das Lösemittel aus der Gruppe ausgewählt ist, die Folgendes umfasst:
a. Bis-(1-butylpentyl)-Decan-1, 10-diyl-Diglutarat;
b. 2-Nitrophenyloctylether;
c. Bis-(2-ethylhexyl)-Phtalat;
d. Dioctyl-Phenyl-Phosphonat; und
- der Träger aus der Gruppe ausgewählt ist, die Folgendes umfasst: Polyvinylchlorid, Zelluloseacetat, Zellulosenitrat.

10. Kreislauf zur Behandlung von Blut, **dadurch gekennzeichnet, dass** er ein Gerät umfasst, das gemäß einem der vorstehenden Ansprüche hergestellt ist.

## Revendications

1. Appareil pour l'anticoagulation régionale du sang, pouvant être utilisé pour réduire la quantité d'ions Ca++ dans le sang qui doit recevoir un traitement dans un circuit pour le traitement de sang, l'appareil comprenant un conduit d'entrée (1) pour le sang collecté depuis le patient, un dispositif pour le traitement du sang (9), un conduit de sortie pour renvoyer le sang traité dans le patient, **caractérisé en ce que** l'appareil comprend une membrane (60) faite d'un matériau sélectivement perméable aux ions Ca++.

2. Appareil selon la revendication 1, **caractérisé en ce qu'**il comprend un moyen (6) pour déterminer une différence de potentiel électrique, positionné et agissant en correspondance avec ledit conduit d'entrée (1) du sang pour déterminer un retrait d'ions Ca++ du sang dirigé vers ledit dispositif de traitement (9).

3. Appareil selon la revendication 2, **caractérisé en ce que** ledit moyen (6) pour déterminer une différence de potentiel électrique comprend une première électrode ou une électrode positive (61) agencée en correspondance avec ledit conduit d'entrée (1) dans une position faisant face ou étant opposée au côté par rapport à ladite membrane (60) et une autre électrode négative (62) symétrique par rapport à la membrane (60) et positionnée de manière opposée à ladite membrane, de manière à ce que les ions Ca++ traversent ladite membrane (60).

4. Appareil selon la revendication 3, **caractérisé en ce que** ladite membrane (60) est agencée interposée entre ledit conduit d'entrée (1) et ledit conduit de sortie (2), ledit moyen (6) pour déterminer une différence de potentiel électrique à condition que la seconde électrode négative (62) soit agencée en correspondance avec le conduit de sortie (2) dans une position faisant face ou étant opposée à ladite membrane (60) de manière à attirer, dans ledit conduit de sortie (2), les ions Ca++ qui traversent ladite membrane (60).

5. Appareil selon la revendication 1, **caractérisé en ce que** ladite membrane (60) est agencée interposée entre ledit conduit d'entrée (1) et ledit conduit de sortie (2), déterminant une communication entre eux pour permettre le transfert d'ions Ca++ depuis le conduit d'entrée (1) vers le conduit de sortie (2).

6. Appareil selon l'une des revendications précédentes, **caractérisé en ce que** le dispositif pour le traitement de sang (9) comprend au moins un composant de l'ensemble consistant en : un hémofiltre, un oxygénateur, des absorbeurs de cytokines, des filtres pour le cholestérol, des cartouches pour la libération de médicaments, un circuit pour une dialyse ou une hémofiltration.

7. Appareil selon l'une des revendications précédentes, où le circuit pour le traitement du sang comprend une pompe (7), **caractérisé en ce que** l'appareil est agencé en amont de la pompe (7).

8. Appareil selon l'une des revendications 1 à 7, où le circuit pour le traitement du sang comprend une pompe (7), **caractérisé en ce que** l'appareil est agencé en aval de la pompe (7).

9. Appareil selon l'une des revendications précédentes, **caractérisé en ce que** la membrane (60) est faite d'un support solide saturé avec un solvant hydrophobe visqueux qui contient un ionophore ou un transporteur ou un porteur, dans lequel :
- l'ionophore ou le transporteur ou le porteur est sélectionné à partir du groupe comprenant :
a. (-)-(R,R)-N,N'-Bis-[11-(éthoxycarbonyl)undécyl]-N,N',4,5-tétraméthyl-3,6-diamide dioxaoctane--, Diéthyl N,N'-[(4R,5R)-4,5-diméthyl-1,8-dioxo-3,6-dioxaoctaméthylène]bis(12-méthylaminododécanoate) ;
b. N,N,N',N'-Tétra[Cyclohexyl] diamide d'acide diglycolique, N,N,N',N'-Tétracyclohexyl-3-oxapentanediamide ;
c. du phosphate de didécyl-calcium ;
d. du borate de potassium tétrakis (4-chlorophényl) ;
- le solvant est sélectionné à partir du groupe comprenant :
a. Bis(1-butylpentyl)décane-1,10-diyl diglutarate ;
b. 2-Nitrophényl octyl éther ;
c. Bis(2-éthylhexyl)phtalate ;
d. dioctyl phényl-phosphonate ; et
- le support est sélectionné à partir du groupe comprenant : du chlorure de polyvinyle, de l'acétate de cellulose, du nitrate de cellulose.

10. Circuit pour le traitement du sang **caractérisé en ce qu'**il comprend un appareil réalisé selon l'une des revendications précédentes.
